Europäisches Patentamt

European Patent Office

Office européen des brevets

(19)

(11) Veröffentlichungsnummer: **0 179 396 B1**

# EUROPÄISCHE PATENTSCHRIFT

(12)

(45) Veröffentlichungstag der Patentschrift: **21.08.91**

(51) Int. Cl.⁵: **A61K 7/16**

(21) Anmeldenummer: **85113165.6**

(22) Anmeldetag: **17.10.85**

(54) Mund- und Zahnpflegemittel.

(30) Priorität: **25.10.84 DE 3439094**

(43) Veröffentlichungstag der Anmeldung:
**30.04.86 Patentblatt 86/18**

(45) Bekanntmachung des Hinweises auf die
Patenterteilung:
**21.08.91 Patentblatt 91/34**

(84) Benannte Vertragsstaaten:
**AT BE CH DE FR GB IT LI NL SE**

(56) Entgegenhaltungen:
DE-A- 2 744 980
US-A- 3 502 587

CHEMICAL ABSTRACTS, Band 81, Nr. 4, 29.
Juli 1974, Seite 223, Zusammenfassung Nr.
16716w, Columbus, Ohio, US; & JP-A-73 37
824 (SUN STAR DENTIFRICE CO., LTD)
14-11-1973

(73) Patentinhaber: **Henkel Kommanditgesellschaft
auf Aktien**
**Postfach 1100 Henkelstrasse 67**
**W-4000 Düsseldorf-Holthausen(DE)**

(72) Erfinder: **Klüppel, Hans-Jürgen, Dr.**
**Begonienstrasse 7**
**W-4000 Düsseldorf(DE)**
Erfinder: **Plöger, Walter, Dr.**
**Holbeinweg 11**
**W-4010 Hilden(DE)**
Erfinder: **Möller, Hinrich, Dr.**
**Schumannstrasse 11**
**W-4019 Monheim(DE)**

## Beschreibung

Gegenstand der Erfindung ist die Verwendung eines Phosphorsäureesters eines alkoxylierten 3- bis 6-wertigen aliphatischen Polyols mit 3 - 12 C-Atomen, der durch Anlagerung von 1 - 15 Mol eines Alkylenoxids mit 2 - 4 C-Atomen an das Polyol und Veresterung wenigstens einer, bevorzugt aller, freien Hydroxylgruppen des Alyoxylats mit Phoshorsäure erhalten wird, oder eines physiologisch verträglichen wasserlöslichen Salzes davon als Wirkstoff gegen die Demineralisation des Zahnschmelzes in Mund- und Zahnpflegemitteln. Eine verbesserte Wirkung gegen Karies findet durch o.g. Substanz statt, mit einer reduzierten Apatitlöslichkeit und Hemmung des Kristallwachstums und Phosphationenaustausch von Hydroxylapatit. Mund- und Zahnpflegemittel sind Produkte, die der Reinigung und Pflege der Zähne, der Mundhöhle und des Rachens dienen. Ihre Aufgabe ist neben der Beseitigung von Mundgeruch und des Zahnbelages die Vorbeugung vor Zahnerkrankungen wie Karies und Parodontose und die Verhinderung der Bildung von Zahnstein.

Es ist bekannt, daß wasserlösliche organische Phosphate eine kariesprophylaktische Wirkung entfalten. Als Zusätze zu Mund- und Zahnpflegemitteln wurden z.B. Mono- und Dinatriumglycerophosphat, Fructose-6-phosphat, Sorbit-6-phosphat, Glucose-1-phosphat und Glucose-6-phosphat vorgeschlagen. Auch Salze von Phosphorsäureestern von Lactose und Saccharose sind als kariostatische Komponenten beschrieben worden. Diese Produkte weisen zwar eine gewisse, die Löslichkeit des Apatit reduzierende und das Kristallwachstum inhibierende Wirkung auf, die Effekte sind jedoch für einen wirksamen Schutz gegen die Demineralisation des Zahnschmelzes nicht ausreichend.

Es wurde nun gefunden, daß Phosphorsäureester von alkoxylierten 3- bis 6-wertigen aliphatischen Polyolen oder deren physiologisch verträgliche wasserlösliche Salze, eine gegenüber den nicht alkoxylierten Polyolphosphaten erheblich gesteigerte Wirkung im Hinblick auf die Senkung der Löslichkeit und die Inhibierung des Kristallwachstums von Hydroxylapatit und des Phosphationenaustausches zwischen Hydroxylapatit und Lösung aufweisen. Diese Produkte eignen sich daher als karieshemmende Wirkstoffe in Mund- und Zahnpflegemitteln.

Gegenstand der Erfindung ist daher die Verwendung von folgender Substanz als Mund- und Zahnpflegemittel mit verbesserter Wirkung gegen Karies: ein Phosphorsäureester eines alkoxylierten 3- bis 6-wertigen aliphatischen Polyols mit 3 - 12 C-Atomen, der durch Anlagerung von 1 - 15 Mol eines Alkylenoxids mit 2 - 4 C-Atomen an das Polyol und Veresterung wenigstens einer, bevorzugt aller, freien Hydroxylgruppen des Alkoxylats mit Phosphorsäure erhalten wird, oder ein physiologisch verträgliches, wasserlösliches Salz davon enthalten.

Die Phosphorsäureester der alkoxylierten Polyole sind bekannt oder nach bekannten Syntheseverfahren herstellbar. Als Polyole können z.B. Glycerin, Erythrit, Trimethylolpropan, Pentaerythrit, Arabit, Xylit, Sorbit, Mannit, Diglycerin, Triglycerin, Dipentaerythrit oder Trimethylolethan verwendet werden. Die Anlagerung von Alkylenoxiden mit 2 - 4 C-Atomen, also Ethylenoxid, Propylenoxid oder Butylenoxid an die Polyole erfolgt nach literaturbekannten Verfahren, also unter Verwendung von basischen Katalysatoren wie z.B. NaOH, KOH, Na-Methylat, Ca-acetat oder sauren Katalysatoren wie z.B. Bortrifluorid, Antimonpentachlorid, Triethyloxoniumfluorborat oder Sn $Cl_4$. Bevorzugt geeignet sind Phosphorsäureester von Anlagerungsprodukten von 1 - 10 Mol Ethylenoxid und/oder Propylenoxid an Glycerin, Erythrit, Trimethylolpropan oder Pentaerythrit. Besonders hohe, das Kristallwachstum von Hydroxylapatit inhibierende Effekte zeigen die Phosphorsäureester von Anlagerungsprodukten von 1 - 7 Mol Ethylenoxid an Glycerin.

Die Veresterung der alkoxylierten Polyole mit Phosphorsäure kann z.B.nach dem in US-PS 4.311.662 beschriebenen Verfahren (für die Herstellung von Korrosionsinhibitoren) durch Umsetzung der alkoxylierten Polyole mit Polyphosphorsäure erfolgen. Dabei ist es zweckmäßig, ein Molverhältnis von 0,5 bis 1,0 Mol $P_2O_5$ pro Hydroxyläquivalent des Alkoxylats einzusetzen. Es werden Produkte erhalten, deren Hydroxylgruppen zu 40 - 100 % in die Phosphorsäureestergruppe überführt sind. Auch US-PS-3,502,587 beschreibt einen solchen Korrosionsinhibitor. Aus keiner der beiden Schriften ist eine Brauchbarkeit zur Hemmung der Demineralisierung des Zahnschmelzes im Mund und in Zahnpflegemitteln herzuleiten. Obwohl auch Produkte, bei denen wenigstens eine der freien Hydroxylgruppen mit Phosphorsäure verestert ist, noch eine gute Wirksamkeit aufweisen, hat sich ein hoher Phosphatierungsgrad, also bevorzugt die Veresterung aller freien Hydroxylgruppen des Alkoxylats als besonders wirksam erwiesen. Die Produkte enthalten neben den organischen Phosphorsäureestern als Nebenprodukt anorganische Phosphate, überwiegend Orthophosphat, das der kariostatischen Wirksamkeit der Produkte nicht abträglich ist und daher im Produkt verbleiben kann.

Der Phosphatierungsgrad kann aus den analytischen Werten für den Gehalt an anorganischem Phosphat und dem Gesamtphosphatgehalt errechnet werden. Die Phosphorsäureester der alkoxylierten Polyole werden nach der Veresterung mit anorganischen oder organischen Basen neutralisiert und auf diese Weise in physiologisch verträgliche, wasserlösliche Salze überführt` Geeignete Salze sind die Alkalisalze, z.B. Na- und/oder K-Salze, die Ammoniumsalze, Mono-, Di- und Triethanol-ammoniumsalze, die Calcium- oder Magnesiumsalze. Weitare zur Salzbildung geeignete Basen sind z.B. Guanidin, Aminosäureester, z.B. die $C_{12}$-$C_{18}$-Fettalkoholester des Lysins, Fettalkyl($C_{12}$-$C_{18}$)-alkoxyalkylamine, 2-Hydroxyalkyl-($C_{12}$-$C_{18}$)-amine und deren Ethylenoxidanlagerungsprodukte. Bevorzugt werden die Natrium- und Kaliumsalze der Phosphorsäureester eingesetzt.

Die Phosphorsäureestern alkoxylierter Polyole gemäß Anspruch 1 können als Mund- und Zahnpflegemittel in den verschiedenen für solche Produkte üblichen Ausführungen, z.B. als Mundwässern, Zahnpasten oder Zahnpulvern Verwendung finden. Der Gehalt an Phosphorsäureestern sollte im Bereich von 0,05 - 5,0 Gew.-% der Zubereitung liegen. Zur Erzielung einer signifikanten karieshemmenden Wirkung sind jedoch in Zahnpasten, Zahnpulvern und Zahngelen Mengen von 0,1 - 2 Gew.-% meist ausreichend. In unverdünnt anzuwendenden Mundwässern können mit 0,05 - 1,0 Gew.-%, in Mundwässern, die vor der Anwendung verdünnt werden, mit entsprechend dem vorgesehenen Verdünnungsverhältnis höheren Konzentrationen ausreichende Effekte erzielt werden.

Mund- und Zahnpflegemittel können darüber hinaus auch in der Form von Kaugummi, Mundpastillen und Zahnbehandlungssalben zubereitet werden. Solche Mundpflegemittel, die gegebenenfalls mehrmals täglich angewandt und zwangsläufig verschluckt werden, können ebenfalls die Phosphorsäureester alkoxylierter Polyole enthalten. In diesen Fällen sollte die Dosierung jedoch nicht über 1 Gew.% der Zubereitung hinausgehen.

Neben den Phosphorsäureestern können die erfindungsgemäßen Mund- und Zahnpflegemittel, die für die jeweilige Zubereitungsform üblichen Träger- und Zusatzmittel enthalten. In Mundwässern ist eine Kombination mit den wäßrig-alkoholischen Lösungen verschiedener Grädigkeit von ätherischen Ölen, Emulgatoren, adstringierenden und tonisierenden Drogenauszügen, zahnsteinhemmenden, antibakteriellen Zusätzen und Geschmackskorrigentien ohne weiteres möglich. Auch oberflächenaktive Substanzen, z.B. anionische, nichtionische, zwitterionische und ampholytische Tenside können in den üblichen Mengen zugesetzt werden.

Unter Zahnpasten oder Zahncremes werden im allgemeinen gelförmige oder pastöse Zubereitungen aus Wasser, Verdickungsmitteln, Feuchthaltemitteln, Schleif- oder Putzkörpern, Tensiden, Süßungsmitteln, Aromastoffen, deodorierenden Wirkstoffen sowie Wirkstoffen gegen Mund- und Zahnerkrankungen verstanden. In die erfindungsgemäßen Zahnpasten können alle üblichen Putzkörper, wie z.B. Kreide, Dicalciumphosphat, unlösliches Natriummetaphosphat, Aluminiumsilikate, Calciumpyrophosphat, feinteilige Kunstharze, Kieselsäuren, Aluminiumoxid und Aluminiumoxidtrihydrat eingesetzt werden.

Bevorzugt geeignete Putzkörper für die erfindungsgemäßen Zahnpasten sind vor allem feinteilige Xerogel-Kieselsäuren, Hydrogelkieselsäuren, Fällungskieselsäuren, Aluminiumoxid-trihydrat und feinteiliges $\alpha$-Aluminiumoxid oder Mischungen dieser Putzkörper in Mengen von 15 - 40 Gew.-% der Zahnpaste. Als Feuchthaltemittel kommen vorwiegend niedermolekulare Polyethylenglykole, Glycerin, Sorbit oder Mischungen dieser Produkte in Mengen bis ca. 50 Gew.-% in Frage. Unter den bekannten Verdickungsmitteln sind die verdickenden, feinteiligen Gelkieselsäuren und Hydrokolloide, wie z.B. Carboxymethylcellulose, Hydroxyethylcellulose, Hydroxypropylguar, Hydroxyethylstärke, Polyvinylpyrrolidon, hochmolekulares Polyethylenglykol, Pflanzengumme wie Traganth, Agar-Agar, Carragheenmoos, Gummiarabicum, Xantham-Gum und Carboxyvinylpolymere (z.B. Carbopol[R]-Typen) geeignet.

Weiterhin können oberflächenaktive Stoffe, bevorzugt anionische schaumstarke Tenside, wie z.B. lineare Natriumalkylsulfate mit 12 - 18 C-Atomen in der Alkylgruppe, Natriumsalze von Alkylpolyglykolethersulfaten mit 12 - 16 C-Atomen in der linearen Alkylgruppe und 2 - 6 Glykolethergruppen im Molekül, von Alkyl-($C_{12-16}$)-benzolsulfonat, linearen Alkan-($C_{12-18}$)-sulfonaten, Sulfobernsteinsäuremonoalkyl-($C_{12-18}$)-estern, sulfatierten Fettsäuremonoglyceriden, sulfatierten Fettsäurealkanolamiden, Sulfoessigsäurealkyl-($C_{12-18}$)-estern, Acylsarkosiden, Acyltauriden und Acylisethionaten mit jeweils 8 - 18 C-Atomen in der Acylgruppe enthalten sein. Auch nichtionische Tenside sind geeignet, z.B. Oxethylate von Fettsäuremono- und -diglyceriden, Fettsäure-Sorbitanestern und Ethylenoxid-Propylenoxid-Blockpolymerisate.

Weitere übliche Zahnpastazusätze sind:

- Konservierungsmittel und antimikrobielle Stoffe wie z.B. p-Hydroxybenzoesäuremethyl-, ethyl- oder propylester, Natriumsorbat, Natriumbenzoat, Bromchlorophen, Phenylsalicylsäureester, Thymol usw.
- Antizahnsteinwirkstoffe, z.B. Organophosphonate wie 1-Hydroxyethan-1.1-diphosphonsäure, 1-Phosphonopropan-1,2,3-tricarbonsäure und andere, die z.B. aus US-PS 3,488,419, DE-OS 2,224,430 und DE-OS 2,343,196 bekannt sind;
- andere karieshemmende Stoffe wie z.B. Natriumfluorid, Natriummonofluorphosphat, Zinnfluorid,
- Süßungsmittel wie z.B. Saccharin-Natrium, Natrium-Cyclamat, Sucrose, Lactose, Maltose, Fructose,

- Aromen wie z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser und anderer natürlicher und synthetischer Aromen,
- Pigmente wie z.B. Titandioxid
- Farbstoffe
- Puffersubstanzen wie z.B. primäre, sekundäre oder tertiäre Alkaliphosphate oder Citronensäure/Natriumcitrat,
- wundheilende und entzündungshemmende Stoffe wie z.B. Allantoin, Harnstoff, Azulen, Kamillewirkstoffe und Acetylsalicylsäurederivate.

Die erfindungsgemäße Verwendung von Phosphorsäureestern alkoxylierter Polyolen als Mund- und Zahnpflegemittel hat nicht nur eine karieshemmende Wirksamkeit, sondern es wird auch die Bildung von Zahnstein erfolgreich entgegengewirkt. In den folgenden Beispielen werden die Herstellung der Phosphorsäureester-Natriumsalze beschrieben, die Wirksamkeit in Hinblick auf die Reduktion der Hydroxylapatitlöslichkeit (ALR), die Inhibierung des Hydroxylapatit-Kristallwachstums (KWI) und die Hemmung der Hydroxylapatit-Phosphationen-Austauschfähigkeit (PIA) nachgewiesen und Anwendungsbeispiele für erfindungsgemäße Mund- und Zahnpflegemittel gegeben:

**Beispiele**

1. Herstellung der Phosphorsäureester-Na-Salze

Die in der folgenden Tabelle aufgeführten Phosphorsäureester Na-Salze wurden nach folgender allgemeiner Herstellvorschrift dargestellt:

1.1 Alkoxylierung

Die Anlagerung von Ethylenoxid (EO) oder Propylenoxid (PO) an das Glycerin erfolgte nach literaturbekanntem Verfahren durch Umsetzung im Druckgefäß in Gegenwart von katalytisch wirkenden Mengen Natriummethylat bei einer Temperatur von 150 - 175°C. Die Produkte wurden durch das Hydroxyl-Äquivalentgewicht charakterisiert, welches aus der Hydroxylzahl (OHZ) errechnet wird.

$$\left[ Val_{(OH)} \;=\; \frac{56110}{OHZ} \right]$$

1.2 Phosphatierung

Zu 169 g (1 Mol $P_2O_5$) Polyphosphorsäure ($P_2O_5$-Gehalt 84 Gew.-%) wurde unter Rühren bei 40 - 50°C 1 Hydroxyläquivalent des Alkoxylats gemäß 1.1 langsam zugesetzt. Nach beendeter Zugabe wurde die Temperatur langsam auf 95 - 100°C erhöht und 3 Stunden unter Rühren auf dieser Temperatur belassen. Anschließend wurde das Reaktionsgemisch mit 350 ml Wasser versetzt und 30 Minuten zum Sieden erhitzt. Danach wurde die braungefärbte Lösung mit 10 g Aktivkohle behandelt, filtriert, abgekühlt und durch Zugabe von 50 %iger Natronlauge auf pH = 7 eingestellt. Die Lösungen wurden ohne Isolierung der Phosphorsäureester-Na-Salze in die folgenden Messungen eingesetzt. Der Wirkstoffgehalt der Lösungen wurde durch Bestimmung des Wassergehalts ermittel (% Wirkstoff = 100 - % $H_2O$).

2. Bestimmung der Reduktion der Apatitlöslichkeit (ALR)

2.1 Blindversuch

In ein Reaktionsgefäß mit 300 ml entsalztem Wasser, thermostatisiert auf 37°C wurde 0,5 g Hydroxylapatitpulver (spez. Oberfläche 60 $m^2$/g, Fa. Merck) gegeben. Der pH-Wert der Suspension wurde mittels einer automatischen Bürette, mit der Milchsäurelösung zugegeben werden kann, auf einen konstanten Wert von pH = 5 gehalten. Die zur pH-Stabilisierung verbrauchte Menge 0,1 m Milchsäurelösung wurde von einem Schreiber registriert. Der nach 2 Stunden registrierte Verbrauch an Milchsäure entsprach der Löslichkeit des unbehandelten Hydroxylapatit (Lu).

4

2.2 Messung

Die Messung wurde analog 2.1 durchgeführt. Vor Zugabe des Hydroxylapatitpulvers wurden 30 mg des zu prüfenden Wirkstoffs aufgelöst. Der nach 2 Stunden registrierte Verbrauch an Milchsäure ensprach der Löslichkeit des behandelten Apatitpulvers (Lb).

Die Reduktion der Apatitlöslichkeit durch den Wirkstoff berechnet sich nach

$$\text{ALR} \left[ \% \right] \quad \frac{(Lu - Lb) \cdot 100}{Lu} \quad \left[ \% \right]$$

Die Ergebnisse der Messungen (ALR) sind in der Tabelle I aufgeführt.

3. Bestimmung der Inhibierung des Kristallwachstums von Hydroxylapatit (KWI)

3.1 Blindversuch

In einem Reaktionsgefäß wurden 400 ml einer 0,0008 molaren Lösung von $KH_2PO_4$ und 45 ml einer 0,012 molaren Lösung von $CaCl_2$ vorgelegt. Diese Lösung wurde durch Titration mit einer 0,05 molaren Lösung von KOH auf einen pH-Wert von 7,4 eingestellt. Nach Erhalt eines über mindestens 30 Minuten stabilen pH-Wertes wurden 100 mg Hydroxylapatitpulver (spez. Oberfläche 60 m²/g, Fa. Merck) zugegeben. Der pH-Wert der Suspension wurde mittels einer automatischen Bürette, mit der 0,05 m KOH-Lösung zugegeben werden kann, auf einem konstanten Wert von 7,4 gehalten. Die zur pH-Statisierung verbrauchte Menge 0,05 m KOH-Lösung wurde van einem Schreiber registriert. Der nach 2 Stunden registrierte Verbrauch an KOH-Lösung (Ku) entsprach der Bildung von Hydroxylapatit (Wachstum der Kristalle der Suspension).

3.2 Messung

Die Messung wurde analog 3.1 durchgeführt. Vor Einstellung des pH-Werts wurden 6 mg des zu prüfenden Wirkstoffs aufgelöst.

Der nach 2 Stunden registrierte Verbrauch an 0,05 m KOH-Lösung (Kb) entsprach der Bildung von Hydroxylapatit (Wachstum der Kristalle in der Suspension) unter dem Einfluß des Wirkstoffs.

Die Inhibierung des Kristallwachstums durch den Wirkstoff berechnet sich nach

$$\text{KWI} \left[ \% \right] = \frac{(K_u - K_b) \cdot 100}{K_u} \quad \left[ \% \right]$$

Die Ergebnisse der Messungen (KWI) sind in Tabelle I aufgeführt.

4. Bestimmung der Hemmung der Phosphationenaustauschfähigkeit von Hydroxylapatit (PIA).

4.1 Blindversuch

In ein Schüttelgefäß mit 250 ml eines mit Hydroxylapatit gesättigten Barbituratpuffers (pH = 7) wurde 1 g Hydroxylapatit (spez. Oberfläche 60 m²/g, Fa. Merck) gegeben und die Suspension 3 Tage bei 20°C zur Gleichgewichtseinstellung geschüttelt. Dann wurde 1 ml einer $Na_2HPO_4$-Lösung in Barbituratpuffer mit einer ³²P-Aktivität von 10 μCi (1 mCi $Na_2HPO_4$ (Aktivität 200 Ci/Mol) in 100 ml Barbituratpuffer) zugegeben. Nach 3 Stunden wurde eine Probe gezogen, über ein Membranfilter filtriert und die Restaktivität $A_{3h}$ bestimmt. Die Hemmung der Phosphationen-Austauschfähigkeit des unbehandelten Hydroxylapatits

$$PIA \quad [\%] \quad = \quad \frac{A_{3h}}{10\,\mu Ci} \cdot 100 \quad [\%]$$

betrug 20 %.

4.2 Vorbehandlung des Hydroxylapatits mit Wirkstoffen

400 mg des Wirkstoffs gelöst in 10 ml Wasser wurden mit 3 g Hydroxylapatitpulver (spez. Oberfläche 60 $m^2$/g, Fa. Merck) und 20 ml eines mit Hydroxylapatit gesättigten Barbituratpuffers (pH = 7) 24 Stunden geschüttelt. Dann wurde der Hydroxylapatit über ein Membranfilter abfiltriert und 2 Stunden bei 50°C getrocknet.

4.3 Messung

1 g des vorbehandelten Hydroxylapatits wurde in gleicher Weise, wie unter 4.1 angegeben, untersucht. Aus dem Aktivitätsverlust der Lösung des vorbehandelten Hydroxylapatits wurde die Hemmung der Phosphationenaustauschfähigkeit PIA % wie folgt berechnet.

$$PIA \quad [\%] \quad \frac{A_{3h}}{10\,\mu Ci} \cdot 100 \quad [\%]$$

Die Ergebnisse der Messungen (PIA) sind in Tabelle I aufgeführt.

6

Tabelle  I

| | Phosphorsäureester-Na-Salz aus: | K W I<br>% Inhibierung des Kristall-wachstums | A R L<br>% Reduktion der Apatit-löslichkeit | P I A<br>% Hemmung der Phos-phationen-austausch-fähigkeit |
|---|---|---|---|---|
| 1 | Glycerin + 1 Mol EO | 53 | 39 | 69 |
| 2 | Glycerin + 2 Mol EO | 44 | 29 | 65 |
| 3 | Glycerin + 3 Mol EO | 46 | 25 | 65 |
| 4 | Glycerin + 7,3 Mol EO | 44 | 14 | 55 |
| 5 | Glyerin + 10 Mol EO | 20 | 16 | 60 |
| 6 | Glycerin + 1 Mol PO | 25 | 29 | 71 |
| 7 | Glycerin + 2 Mol PO | 34 | 32 | 69 |
| 8 | Glycerin + 3 Mol PO | 18 | 18 | 61 |
| 9 | Glycerin + 6 Mol PO | 21 | 25 | 58 |
| 10 | Glycerin + 10 Mol PO | 21 | 10 | 51 |
| 11 | Glycerin + 13 Mol PO | 11 | 6 | 56 |
| Vergleichssubstanzen | | | | |
| | Glycerin-Phosphat (hergestellt nach 1.2) | 27 | 18 | 69 |
| | ß-Glycerinphosphat | 0 | 6 | 21 |
| | DL- $\alpha$-Glycerinphosphat | 0 | 1 | 24 |

5. Anwendungsbeispiele

5.1 Zahnpasta

| | | |
|---|---:|---|
| Fällungskieselsäure [1] | 18 | Gew.% |
| Verdickungskieselsäure (pyrogen) [2] | 0,8 | Gew.% |
| Glycerin + 1 Mol EO-Phosphat (nach Beisiel 1) | 1,0 | Gew.% |
| Sorbit | 17,5 | Gew.% |
| Glycerin | 17,5 | Gew.% |
| Carboxymethylcellulose [3] | 0,9 | Gew.% |
| Natrium-Laurylsulfat [4] | 2,0 | Gew.% |
| Natriumfluorid | 0,22 | Gew.% |
| Saccharin-Natrium | 0,2 | Gew.% |
| Aromaöle | 1,0 | Gew.% |
| Wasser, Konservierungsmittel | ad 100 | Gew.% |

5.2 Mundwasser

| | | |
|---|---:|---|
| Ethylalkohol (96 Vol%) | 10 | Gew.% |
| Polyoxyethylensorbitanmono-laurat [5] | 0,4 | Gew.% |
| Aromaöl | 0,3 | Gew.% |
| Sorbit (70 %ige wässr. Lsg.) | 8,0 | Gew.% |
| p-Hydroxybenzoesäure-methylester | 0,16 | Gew.% |
| Glycerin + 1 Mol EO-phosphat (nach Beispiel 1) | 0,1 | Gew.% |
| Saccharin-Natrium | 0,1 | Gew.% |
| Wasser, Farbstoffe | ad 100 | Gew.% |

[1] Sident(R) 12DS, Fa. Degussa
[2] Aerosil(R) 200, Fa. Degussa
[3] Relatin(R) 100 S8, Fa. Henkel KGaA
[4] Texapon K1296, Fa. Henkel KGaA
[5] Tween(R) 20, Atlas Chemie

**Patentansprüche**

1. Verwendung eines Phosphorsäureesters eines alkoxylierten 3- bis 6-wertigen aliphatischen Polyols mit 3 - 12 C-Atomen, der durch Anlagerung von 1 - 15 Mol eines Alkylenoxids mit 2 - 4 C-Atomen an das Polyol und Veresterung wenigstens einer, bevorzugt aller, freien Hydroxylgruppen des Alkoxylats mit Phosphorsäure erhalten wird, oder eines physiologisch verträglichen wasserlöslichen Salzes davon als Wirkstoff gegen die Demineralisation des Zahnschmelzes in Mund- und Zahnpflegemitteln.

2. Verwendung nach Anspruch 1, dadurch gekennzeichnet, daß der Phosporsäureester eines Anlagerungsproduktes von 1 - 10 Mol Ethylenoxid und/oder Propylenoxid an Glycerin, Trimethylolpropan oder Pentaerythrit oder das Natrium- oder Kaliumsalz davon eingesetzt wird.

3. Verwendung nach Anspruch 1 oder 2, dadurch gekennzeichnet, daß der Phosphorsäureester eines Anlagerungsproduktes von 1 - 7 Mol Ethylenoxid an Glycerin eingesetzt wird.

4. Verwendung nach Anspruch 1 - 3, dadurch gekennzeichnet, daß der Phosphorsäureester in einer Menge von 0,05 bis 5,0 Gew.-% der Zubereitung neben üblichen Komponenten von Mund- und Zahnpflegemitteln eingesetzt wird.

5. Zahnpaste in Form einer pastösen Zubereitung aus Wasser, Feuchthaltemitteln, Verdickungsmitteln, Schleif- und Putzkörpern, Geschmacks- und Aromazusätzen, deren Schleif- und Putzkörper ganz oder überwiegend aus feinteiligen Xerogelkieselsäuren, Hydrogelkieselsäuren, Fällungskieselsäuren, Aluminiumoxid-trihydrat und/oder feinteiligem Aluminiumoxid besteht, dadurch gekennzeichnet, daß als Wirkstoffe ein Phosphorsäureester der in Anspruch 1 - 3 definierten Art in einer Menge von 0,1 - 2,0 Gew.-% der gesamten Zubereitung enthalten ist.

6. Mundwasser in Form einer wäßrigen oder wäßrig-alkoholischen Lösung mit üblichen Geschmacks- und Aromazusätzen, dadurch gekennzeichnet, daß als Wirkstoff ein Phosphorsäureester der in Anspruch 1 - 3 definierten Art in einer Menge von 0,05 - 1 Gew.-%, bei verdünnt anzuwendenden Mundwässern in entsprechend höherer Konzentration, enthalten ist.

## Claims

1. The use of a phosphoric acid ester of an alkoxylated, trihydric to hexahydric aliphatic polyol containing 3 to 12 carbon atoms, which is obtained by addition of 1 to 15 mol of a $C_{2-4}$ alkylene oxide onto the polyol and esterification of at least one and preferably all the free hydroxyl groups of the alkoxylate with phosphoric acid, or a physiologically compatible water-soluble salt thereof as an active substance against the demineralization of dental enamel in oral and dental hygiene preparations.

2. The use claimed in claim 1, characterized in that the phosphoric acid ester of an adduct of 1 to 10 mol of ethylene oxide and/or propylene oxide with glycerol, trimethylol propane or pentaerythritol or the sodium or potassium salt thereof is used.

3. The use claimed in claim 1 or 2, characterized in that the phosphoric acid ester of an adduct of 1 to 7 mol of ethylene oxide with glycerol is used.

4. The use claimed in claims 1 to 3, characterized in that the phosphoric acid ester is used in a quantity of 0.05 to 5.0% by weight of the preparation in addition to standard components of oral and dental hygiene preparations.

5. A toothpaste in the form of a paste-like preparation of water, humectants, thickeners, abrasives and polishes, flavour correctants and flavourings, of which the abrasive and polish consists completely or predominantly of finely particulate xerogel silicas, hydrogel silicas, precipitated silicas, aluminium oxide trihydrate and/or finely particulate aluminium oxide, characterized in that it contains as active component a phosphoric acid ester of the type claimed in claims 1 to 3 in a quantity of 0.1 to 2.0% by weight of the preparation as a whole.

6. A mouthwash in the form of an aqueous or aqueous-alcoholic solution containing the usual flavour correctants and flavourings, characterized in that it contains as active component a phosphoric acid ester of the type defined in claims 1 to 3 in a quantity of 0.05 to 1% by weight or, for mouthwashes used in dilute form, in correspondingly higher concentrations.

## Revendications

1. Mise en oeuvre d'un ester phosphorique d'un polyol aliphatique trivalent à hexavalent alcoxylé avec 3 à 12 atomes C qui est obtenu par addition de 1 à 15 moles d'un alkylèneoxyde avec 2 à 4 atomes C au polyol et estérification d'au moins un radical hydroxyle libre, et de préférence de tous ceux-ci, de l'alcoxylat avec de l'acide phosphorique ou d'un sel hydrosoluble compatible physiologiquement de celui-ci comme agent actif contre la déminéralisation de l'émail dentaire dans les produits d'hygiène buccale et dentaire.

2. Mise en oeuvre selon la revendication 1 caractérisée en ce que l'ester phosphorique d'un produit d'addition de 1 à 10 moles d'éthylèneoxyde et/ou de propylèneoxyde à la glycérine, au triméthylolpropane ou au pentaérythritol ou un sel de sodium ou de potassium de ceux-ci est utilisé.

3. Mise en oeuvre selon la revendication 1 ou 2 caractérisée en ce que l'ester phosphorique d'un produit d'addition de 1 à 7 moles d'éthylènoxyde à la glycérine est utilisé.

4. Mise en oeuvre selon la revendication 1 à 3 caractérisée en ce que l'ester phosphorique est utilisé dans une quantité de 0,05 à 5,0 % en poids de la préparation outre les composants classiques des produits d'hygiène buccale et dentaire.

5. Dentifrice sous forme d'une préparation pâteuse à base d'eau, d'agents hydratants, d'agents épaississants, de corps abrasifs et nettoyants, de correcteurs de goût et d'arômes, dont les corps abrasifs et nettoyants se composent totalement ou essentiellement d'acides siliciques en xérogel à petites particules, d'acides siliciques en hydrogel, d'acides siliciques précipités, de trihydrate d'alumine et/ou d'alumine en petites particules, caractérisé en ce qu'un ester phosphorique du type défini dans les revendications 1 à 3 est utilisé dans une quantité de 0,1 à 2,0 % en poids de l'ensemble de la préparation comme substance active.

6. Collutoire sous forme d'une solution aqueuse ou alcoolique aqueuse avec les correcteurs de goût et arômes usuels, caractérisé en ce qu'un ester phosphorique du type défini dans les revendications 1 à 3 est utilisé comme substance active dans une quantité de 0,05 à 1 % en poids ou, en cas de collutoires à utiliser sous forme diluée, dans des concentrations proportionnellement plus élevées.